# EUROPEAN PATENT APPLICATION

(11) **EP 0 552 916 A1**
(43) Date of publication of application: **28.07.1993**
(21) Application number: 93300327.9
(22) Date of filing: 19.01.1993
(51) Int. Cl.: A61B 5/087, G01F 1/36, G01F 1/38, G01F 1/44

(54) **A sidestream flow sensor for spirometry**

(30) Priority: 21.01.1992 US 823193
(71) Applicant: PURITAN-BENNETT CORPORATION, Kansas City, Kansas (US)
(72) Inventor: Neville, Dee J., Simi Valley, California 93065 (US)
(74) Representative: Mayes, Stuart David

(57) **Abstract**

An improved spirometer for measuring the flow rate of breaths includes a mainstream conduit having a fixed or variable restrictor (14) formed therein for producing a pressure differential across the restrictor, and one or more sidestream conduits having a first end (18) in fluid communication with the mainstream conduit, upstream of the restrictor, and a second end (20) in fluid communication with the restrictor. A thermal flow sensor (24) is disposed within each sidestream conduit, and includes a heater element (76) and a pair of temperature sensors (78,80) located upstream and downstream of the heater element connected in a bridge configuration to generate an analog signal for output to a data processing system to measure the flow rate of breath in the tube.

## Description

### BACKGROUND OF THE INVENTION

### Field of the Invention

This invention relates generally to a method and apparatus for measuring flow and, more particularly, to an apparatus which measures patient airflow in a ventilator used to provide respiration support to a patient.

### Description of the Related Art

Among the techniques that have been utilized for measuring the volume of air moved into or out from the lungs of a patient being supported by a respirator are: (1) a hot film element, inserted into the patient's airstream in conjunction with a thermistor; (2) a plurality of hot wires inserted into the patient's airstream; and (3) a flow linearizer or restrictor inserted into the patient's airstream to provide a pressure drop proportional to the flow rate. The hot film and hot wire devices have the advantage of providing a very sensitive fast response to extremely low flow rates. Unfortunately, exposed hot film and hot wire devices are vulnerable to airborne particulates which may coat or damage the heated element. Sensors incorporating a linearizer element generally have a rather limited range of flow rates for which the sensor response is linear. Furthermore, the linearized sensors typically require a high quality, relatively expensive pressure transducer and tend to be pneumatically noisy.

Another limitation of such conventional devices is the placement of an obstruction in the patient airway which is thus vulnerable to the accumulation of mucus, medicants, and moisture condensation, which can result in flow measurement errors. These adverse environmental conditions, combined with the fragility of hot film and hot wire devices, contribute to the tendency of such sensors to prematurely deteriorate and cease to function. Furthermore, such sensors tend to be difficult to clean, and in general, are usually too expensive to be readily disposable.

Various attempts have been made to reduce the vulnerability of such devices. For example, shrouds have been introduced to shield the hot film and hot wire elements. Other configurations utilized in attempts to resolve these problems include instruments having a mainstream restrictor and a thermal sensing flow tube instead of a pressure transducer. However, such devices tend to respond slowly to variations in the measured airflow due to their enclosed configuration. Another approach has been the use of a mainstream restrictor in fluid communication with a sidestream or bypass system, with a paddle mounted on a movable lever extending from a strain gauge positioned within the sidestream. This particular device, however, is mechanically delicate, and is susceptible to many of the same problems described above.

Hence, those concerned with the development and use of flow sensors have long recognized the need for a sensitive, rugged and low cost flow sensing system for accurately measuring patient flow rates. Embodiments of the present invention offer a solution to these requirements.

### SUMMARY OF THE INVENTION

Briefly, and in general terms, embodiments of the present invention provide a new and improved method and apparatus for sensitive and accurate measurement of air flow to and from a patient intubated on a respirator. One embodiment is particularly useful as an improved spirometer which diverts a proportional amount of air from a mainstream conduit along a sidestream conduit for measurement.

In accordance with a particular embodiment of the invention, at least one sidestream flow path is provided in fluid communication with a mainstream flow conduit between the patient and a respirator. The mainstream conduit preferably contains a restrictor which acts to divert a portion of the flow through the sidestream flow path. The sidestream flow through the sidestream flow path is measured to provide an indication of flow in the mainstream flow conduit. A plurality of appropriately sized sidestream flow paths and associated sensors may be used to accurately measure mainstream flow circuit flow rates over a range of flow rates. The flow sensors in the sidestream flow paths are preferably thermal bridge type sensors each including a heater element and a pair of temperature sensors upstream and downstream from the heater element and arranged in a bridge configuration. A computer and switching means may be used to sense the flow rate through at least one sidestream sensor and determine the flow rate of the mainstream flow conduit on the basis of the outputs of the sidestream sensor. In one embodiment of the invention, a general purpose computer may be programmed with tabular information of sidestream flow sensor outputs as a function of main flow conduit flow rates, thereby allowing a calculation of mainstream conduit flow rates on the basis of sidestream flow sensor outputs.

In one preferred embodiment of the present invention, which is included by way of example and not necessarily by way of limitation, the mainstream conduit includes a venturi. A small portion of the air flowing through the mainstream conduit is diverted through a sidestream sensor subsystem. The sidestream sensor subsystem includes at least one sidestream conduit having a cross-sectional area significantly less than the mainstream conduit. One end of the sidestream conduit has a positive pressure port in fluid communication with the mainstream conduit upstream from a venturi throat. The other end of the sidestream conduit has a negative pressure port in fluid communication with the mainstream conduit at the venturi throat. In such an arrangement, the flow of gas through the sidestream conduit is approximately proportional to the flow through the mainstream conduit. A sidestream sensor disposed within the sidestream conduit generates an output signal for later conditioning and translation into various patient airflow parameters. A small bacteria type filter may also be positioned in the sidestream conduit. A bias airflow system may be used to maintain a constant flow through the venturi throat, reducing the effects of moisture condensation within the mainstream and sidestream conduits. The mainstream conduit may be arranged vertically to prevent the accumulation of fluids in the mainstream conduit and the sidestream conduit.

In an alternative embodiment for measuring patient airflow the invention includes a mainstream conduit and sidestream sensor subsystem, and an additional outer conduit surrounds the mainstream conduit. The incoming moisture laden gas is directed against the exterior of the mainstream conduit for countercurrent heat exchange with the mainstream conduit. Moisturee carried within the incoming moisture laden gas cools and condenses upon the exterior walls of the mainstream conduit. The dried air then flows through the sensor system and also through the vertical venturi as in the other embodiment. Condensation collects on the exterior of the mainstream conduit, and, with time, coalesces to collect within a reservoir at the bottom of the apparatus.

In still another preferred embodiment of the present invention, the sidestream system may include a plurality of sidestream conduits of various cross-sectional areas, each with an associated thermal bridge type sensor operative to sense the flow through its sidestream. Through design and experimentation, the sidestream conduits may be sized to provide indications of flow in the main conduit over a range of flow rates beyond the range of a single sidestream. The outputs of the sidestream flow sensors may be provided to an indicator means which derives a measure of flow rate through the mainstream conduit from the output of the sidestream sensors. One form of indicator means for such a purpose may include a computer including microprocessor means programmed to relate the output of the flow sensors to the main conduit flow rate. In practice, it has been found that by utilizing a plurality of sidestreams of various cross-sectional areas, the measurement may be reliably obtained over a range of mainstream flow rates well beyond the range of a single sidestream flow sensor. When using such a scheme, the output of the sidestream sensors may be determined as a function of the mainstream conduit flow rate and such data stored as a "table look up" function of the type well known in the computer arts to determine main conduit flow rates from the sidestream sensor outputs. Alternatively, the information relating sidestream sensor output to the mainstream flow rates may be reduced to an algorithm incorporated in the computer software, thereby allowing the direct calculation of the flow rates from the sidestream sensor outputs. In still another embodiment of the invention, the relationship of the outputs of the sidestream flow sensors to the mainstream conduit flow may be incorporated in an electrical circuit other than a digital computer which operates to provide an indication of mainstream flow rates based upon the output signals from the sidestream flow sensors.

From the above, it may be seen that embodiments of the present invention provide an improved method and apparatus for the measurement of flow in a ventilator system with a primary airway for conveying breathing gases to and from a patient. These and other objects and advantages of the invention will become apparent from the following more detailed description, when taken in conjunction with the accompanying drawings of the illustrative embodiment.

### BRIEF DESCRIPTION OF THE DRAWINGS

FIGURE 1 is a perspective view of a flow sensing system in accordance with an embodiment of the present invention shown in combination with a ventilator;
FIGURE 2 is a schematic, cross-sectional view of the flow sensing system of Fig. 1;
FIGURE 3 is a perspective view of the flow sensing system similar to that of Fig. 1 showing a cut away view of a portion of the system;
FIGURE 4 is a graph showing the desired venturi airflow characteristics as expressed in a relationship between differential pressure (ΔP) and patient airflow (Q);
FIGURE 5 is an enlarged fragmentary sectional view of a detachable segment of the flow sensing system of Fig. 1;
FIGURE 6 is an enlarged, fragmentary sectional view of another embodiment of the detachable segment of the flow sensing system of Fig. 1;
FIGURE 7 is an enlarged fragmentary perspective view of the mainstream conduit in combination with a bias flow embodiment of the flow sensing system of Fig. 1;
FIGURE 8 is a schematic view of the sidestream flow path of the flow sensing system of Fig. 1;
FIGURE 9 is a graph showing the relationship between sidestream flow (q) and venturi pressure differential (ΔP);
FIGURE 10 is a graph showing the relationship between sidestream flow (q) and patient airflow (Q);
FIGURE 11 is a schematic diagram of the thermal bridge type of flow sensor of the flow sensing system of Fig. 1;
FIGURE 12 is a graph showing the relationship between the thermal bridge type sensor DC analog signal and sidestream flow;
FIGURE 13 is a graph showing the relationship between the thermal bridge type sensor DC analog signal and patient airflow;
FIGURE 14 is an alternative embodiment of the flow sensing system of Fig. 1;
FIGURE 15 is a fragmentary cross-sectional view of a further embodiment of the flow sensing system of Fig. 1;
FIGURE 16 is a graph showing the relationship between the thermal bridge type sensor DC analog signal and sidestream flow of the flow sensing system of Fig. 15;
FIGURE 17 is a graph showing the relationship between the thermal bridge type sensor DC analog signal and patient airflow of the flow sensing system of Fig. 15;
FIGURE 18 is a schematic, cross-sectional view of another embodiment of the flow sensing system of the invention incorporating a variable orifice mainstream restrictor and a plurality of sidestream conduits;
FIGURE 19 is a schematic, cross-sectional view of the flow sensing system similar to that of Fig. 18, with a single sidestream and a filter;
FIGURE 20 is a graph showing the generally linear relationship between the sidestream flow and a mainstream variable orifice pressure differential (ΔP);
FIGURE 21 is a graph showing the relationship between the variable mainstream orifice pressure differential (ΔP) and mainstream patient airflow;
FIGURE 22 is a graph showing the relationship between the sidestream flow and patient airflow through a mainstream variable orifice;
FIGURE 23 is a graph showing the relationship between the thermal bridge type sensor DC analog signal and sidestream airflow; and
FIGURE 24 is a graph showing the relationship between the thermal bridge type sensor DC analog signal and patient airflow through a variable orifice restrictor.

### DETAILED DESCRIPTION OF THE PREFERRED EMBODIMENTS

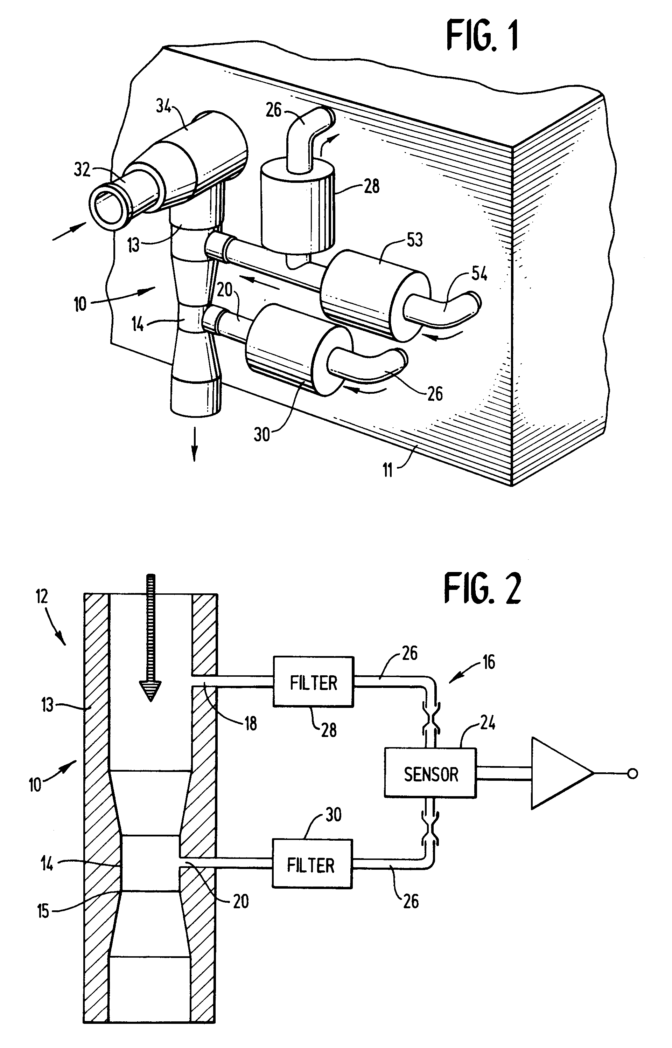

As shown in the exemplary drawings, which are included for the purposes of illustration and not by way of limitation, embodiments of the present invention provide an improved sidestream flow sensor for accurate and reliable measurement of air flow to and from a patient intubated on a respirator. Referring to Fig. 1, a flow sensing system 10 in accordance with an embodiment of the invention is shown in a typical position adjacent a respirator or ventilator apparatus 11 and is provided with a novel construction which sensitively, accurately, and reliably determines parameters of a patient intubated on the ventilator. As will be explained hereinafter in greater detail, the flow sensing system preferably includes at least a portion of the mainstream conduit 13 conducting airflow between the ventilator and the patient. A narrowed restrictor portion 14 is preferably formed in the mainstream conduit to divert a portion of the patient airflows through a sidestream flow sensor subsystem 16 having a positive pressure port 18 connected to an unrestricted portion of the mainstream conduit, and a negative pressure port connected to the narrowed restrictor portion. Flow sensor means 14 is preferably provided in the sidestream conduit 26 connected for fluid communication between the positive and negative ports, and filters 26 and 28 may be provided in the sidestream conduit on either side of the flow sensor means, to filter out small bacteria or other small particulates, and thus minimize contamination of the sensor means. A bias flow subsystem having a bias flow conduit 54 and associated filter 53 may also be provided to insure that there will be a constant minimum flow of gas to the sidestream sensor subsystem.

As can be seen more easily in the simplified schematic view of Fig. 1 shown in Fig. 2, the flow sensing system 10 includes a mainstream subsystem 12 including at least a portion of a mainstream conduit 13 with a narrowed restrictor portion 14 formed therein. While in the presently preferred embodiment, the mainstream restrictor 14 is in the form of a gradually narrowing venturi throat 15, still further embodiments may incorporate a mainstream restrictor in a form other than a venturi restriction. Indeed, it is contemplated that other forms of mainstream conduit restrictors, fixed or variable, may be utilized. For example, capillary tubes, straight tubes, elbows or filter or screen materials may be incorporated as mainstream restrictors. The flow path of the sidestream sensor subsystem 16 is in fluid communication with the mainstream conduit 13 at a positive port 18 at a location in an unrestricted portion of the mainstream conduit removed from the mainstream restrictor 14 and at negative port 20 within the mainstream restrictor 14. In a preferred embodiment, the negative port 20 is positioned at the most restricted or narrowest portion of the mainstream restrictor 14. Flow sensor means 24 is disposed within the sidestream conduit 26 to generate an output signal indicative of the airflow therethrough.

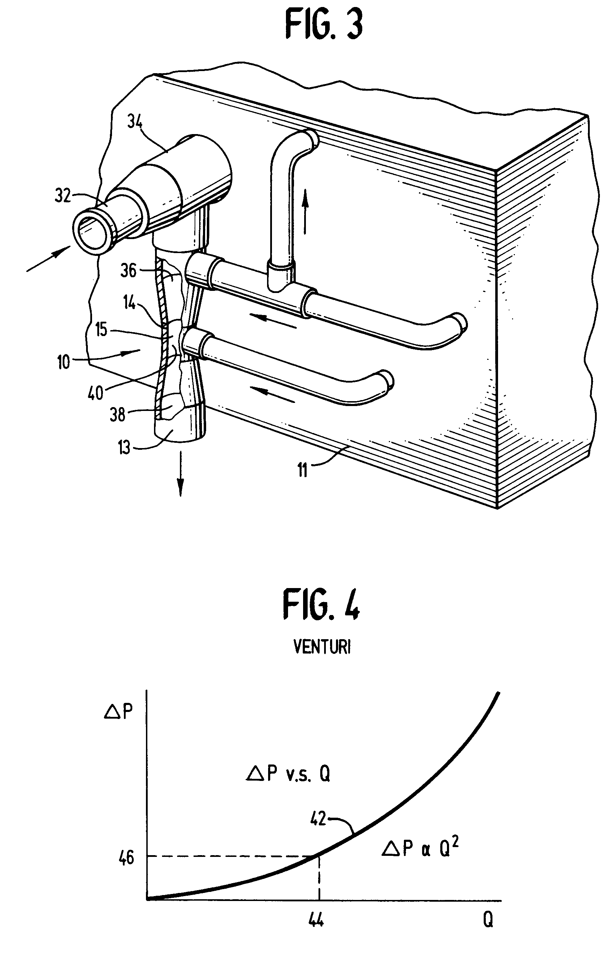

With reference to Fig. 3, showing a simplified perspective view of the system of Fig. 1 without the conduit filters, an external connecting conduit 31 preferably places a patient (not shown) in fluid communication with an exhalation check valve 34 of the ventilator in fluid communication with mainstream conduit 13, which in one preferred embodiment is in a generally vertical orientation. The mainstream restrictor 14, when in the form of a venturi 15, has first and second gradually tapering portions 36 and 38 having outside ends connected to the unrestricted mainstream conduit, and narrowed inside ends connected to the most constricted portion or orifice 40. The venturi 15 is dimensioned to provide a differential pressure (ΔP) approximately proportional to the square of the patient's airflow (hereinafter "Q") across the venturi. Fig. 4 shows a curve 42, representing the relationship between differential pressure ΔP and Q. Thus, when the patient has an airflow Q represented by point 44 on curve 42, a differential pressure represented by the point 46 is produced across the venturi.

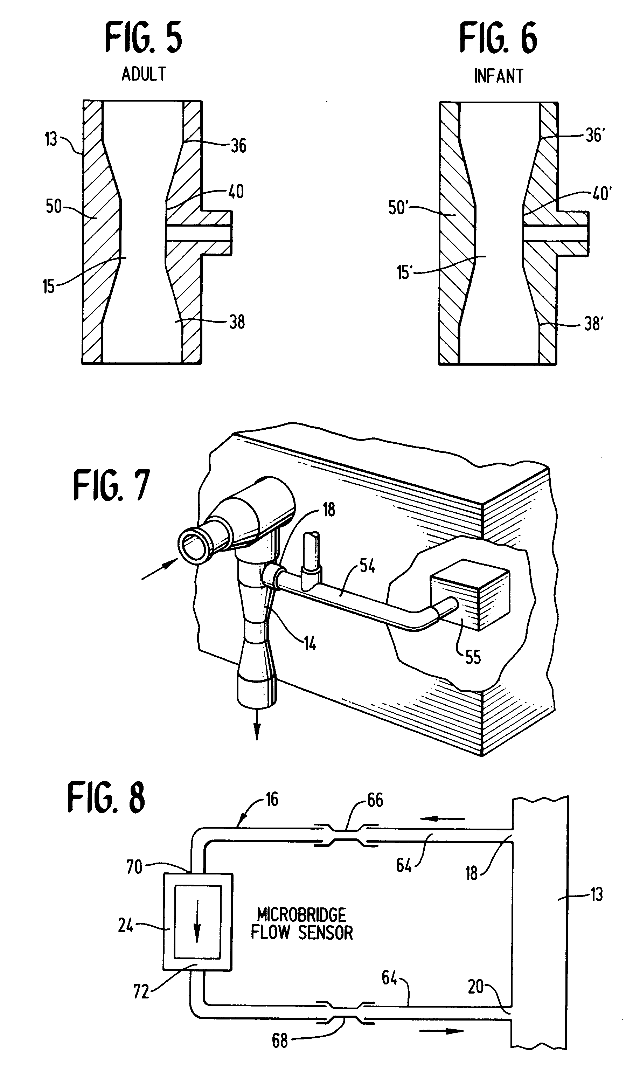

In one preferred embodiment illustrated in Figs. 5 and 6, the venturi 15 may be advantageously formed within a detachable segment 50 of the mainstream conduit 13 having a venturi 15 connectable to the negative port 20 of the sidestream flow sensor subsystem, which permits selection of an appropriately sized venturi constriction as required by the particular patient. The detachable segment 50 is preferably constructed of material which is either disposable or reusable. If it is formed of reusable material, detachable segment 50 should be sterizable by any suitable method. In general, there will be two preferred detachable segment 50 embodiments. The ventilator 11 may be keyed for positive identification of which size is to be installed.

In one particular embodiment for the adult patient referring now to Fig. 5, the detachable segment 50 includes a venturi 15 having venturi tapered portions 36 and 38 with an inside diameter at their widest part of about 5/8ths of an inch. The constricted portion or orifice 40 will have an inside diameter of about 3/8ths of an inch. Preferably, the length of the detachable segment 50, in this embodiment, is about three inches. An outside diameter of about 7/8ths of an inch is sufficient. In this configuration, backpressure (resistance) should be leas than six centimeters of H₂O at 180 liters per minute (hereinafter "lpm"). A differential pressure ΔP across the venturi of approximately fifteen centimeters of H₂O will be generated by an airflow of approximately 180 lpm through a venturi with such a configuration.

Alternatively, in a pediatric embodiment shown in Fig. 6, the detachable segment 50' includes a venturi 15 modified to provide about one-fourth of the adult flow range. The pediatric detachable segment 50' has venturi tapered portions having an inside diameter at their widest part of approximately 5/8ths of an inch, the same as in the adult venturi. The pediatric venturi orifice 40' preferably has an inside diameter of about 3/16ths of an inch. The length and the outside diameter of the pediatric segment 50' should be the same as in the adult venturi detachable segment 50 to allow for easily interchangeability. In this configuration, the backpressure (resistance) should be less than six centimeters of H₂O at forty-five lpm and the differential pressure (signal) should be about fifteen centimeters of H₂O at forty-five lpm.

In one preferred embodiment illustrated in simplified form in Fig. 7, a bias flow system includes a bias flow conduit 54 in fluid communication with the positive port 18 to effect a continuous minimal flow from a source 55 of pair through the sidestream flow path, even when no air is being forced through the mainstream venturi by the patient. For example, when the bias air supply has a flow rate represented by a greater amount, i.e., greater than the flow rate at some minimal value, an output signal of some measurable magnitude is produced continuously by the flow sensor, even though no air flow is being produced by the patient. Thus, when the patient exhales through the venturi, the gas flow sensor means always produces an output signal of a minimum threshold. The minimum threshold signal due to the bias airflow may thus be subtracted from the output signal indicating the actual flow, to give a measurement of the flow rate actually produced by the patient.

This bias airflow of dry air advantageously mixes with the patient's moist breath to reduce the relative humidity within the venturi tube and to thereby reduce condensation formation inside the venturi tube. Generally without bias air, the venturi 15 will get wet or stay wet depending on the flow rate or nebulization. The perceived error in the determined flow parameters at medium and high flows, e.g., from about 100 to about 200 lpm, due to water droplets forming or blowing through the venturi orifice 40, seems to be negligible (less than 2%) and can probably be compensated for by calibration. However, fogging may occur at low flows, e.g., from about 1 to about 10 lpm, and with nebulization, a heavy build-up in the throat and an attending error of up to 10% may occur.

The bias flow also provides other benefits. For example, the need for heating of any part of the flow sensor system 10 to reduce condensation is reduced. The bias flow helps keep the positive pressure port 18 dry, and helps keep the patient's air out of the sidestream tubing circuit and sensor. In addition, filters used within the sensor sidestream subsystem remain drier since primarily dry bias blow air flows therethrough.

In addition to keeping condensation from forming on the venturi 15, and in the sidestream sensor system 16, the bias flow improves the quality of the output of the sensor. The bias air supply provides for more equal input and output signals for the same rates of inhalation and exhalation than would be produced if no bias air supply were present. In addition, the bias air supply improves the low range sensitivity of the 3/8ths inch venturi from a minimum of 3 lpm to minimum of 1 lpm. The error due to zero drift at this point is approximately plus or minus 2 lpm, but with auto zero, the drift error may be reduced to plus or minus 1/2 lpm. With a 12 bit a/d and an analog input range of 1.0 millivolt ("mv") to about 4.0 volt, the resolution at this minimum point will be plus or minus 1/2 lpm. The net minimum is then plus or minus 1 lpm. In a preferred embodiment, the bias flow should be about 5 lpm plus or minus 25%, with a stability of plus or minus 5%. This provides a minimum resolution of 1 lpm at the low flow in the adult venturi embodiment, and better than .25 lpm at low flow in pediatric venturi embodiment.

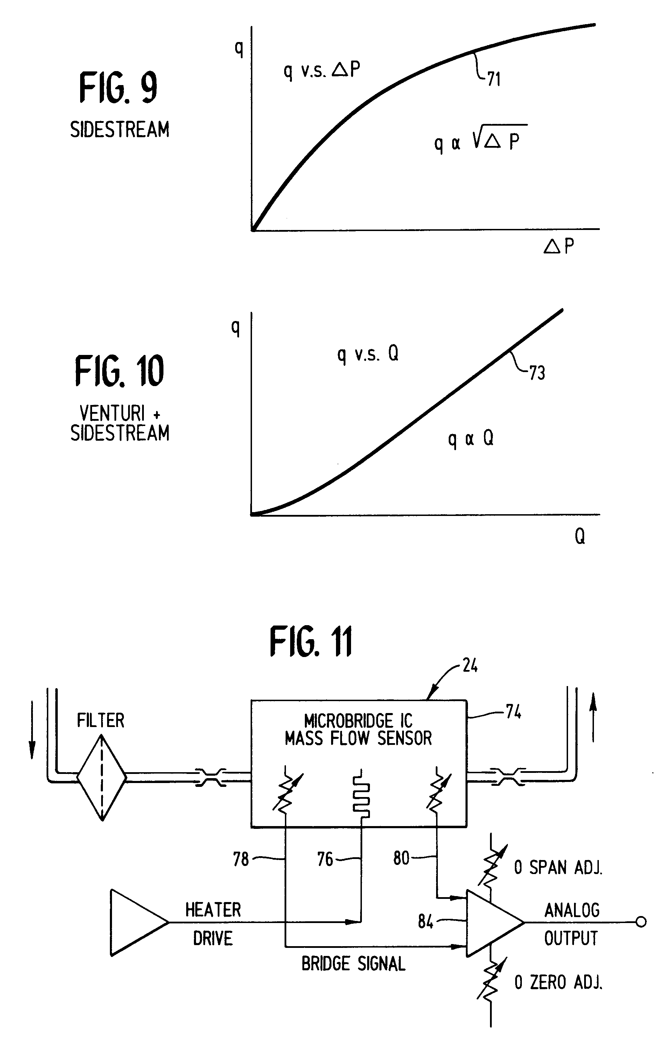

As can best be seen in Fig. 8, the sidestream subsystem 16 is in fluid communication with the mainstream conduit 13 through the positive and negative ports 18 and 20, respectively, and through sidestream connecting tubing 64, having a cross-sectional area significantly less than that of the mainstream conduit, extending from the positive port 18 and negative port 20 to be in fluid communication through sensor means 24. The sidestream connecting tubing may for example be silicone tubing having approximately an 1/8th of an inch inside diameter. First and second precision output scaling restrictors 66 and 68, respectively, preferably in the form of a venturi, and having about a 10/1000ths of an inch inside diameter, are placed symmetrically within the sidestream connecting tubing 64 or either side of the sensor means, and are in fluid communication with the inlet 70 and outlet 71, respectively, of the flow sensor means 24. The output scaling restrictors allow scaling of the flow to the sensor means, and filtering of pneumatic noise from the flow sensor subsystem. Maintaining symmetry in both lengths of the sidestream connecting tubing 64 and symmetry in the positioning of the restrictors 66 and 68 facilitates good common mode rejection of pneumatic noise. for example, a reduction in pneumatic noise of about 10 to about 100 times has been noted. In one particularly preferred embodiment, the output scaling restrictors of the sidestream sensor subsystem allow scaling of the sidestream flow ("q") to be generally proportional to the square root of venturi differential pressure ΔP, i.e., q ∝ √ΔP. In Fig. 9 curve 71 represents this preferred relationship between sidestream flow q and venturi differential pressure ΔP. Sidestream flow q, will also generally be directly proportional to the patient airflow (Q), and as is shown in Fig. 10, which depicts curve 73, representing the relationship between sidestream flow and patient airflow. In this embodiment, sidestream airflow, q, should be approximately the patient airflow Q divided by 1,000.

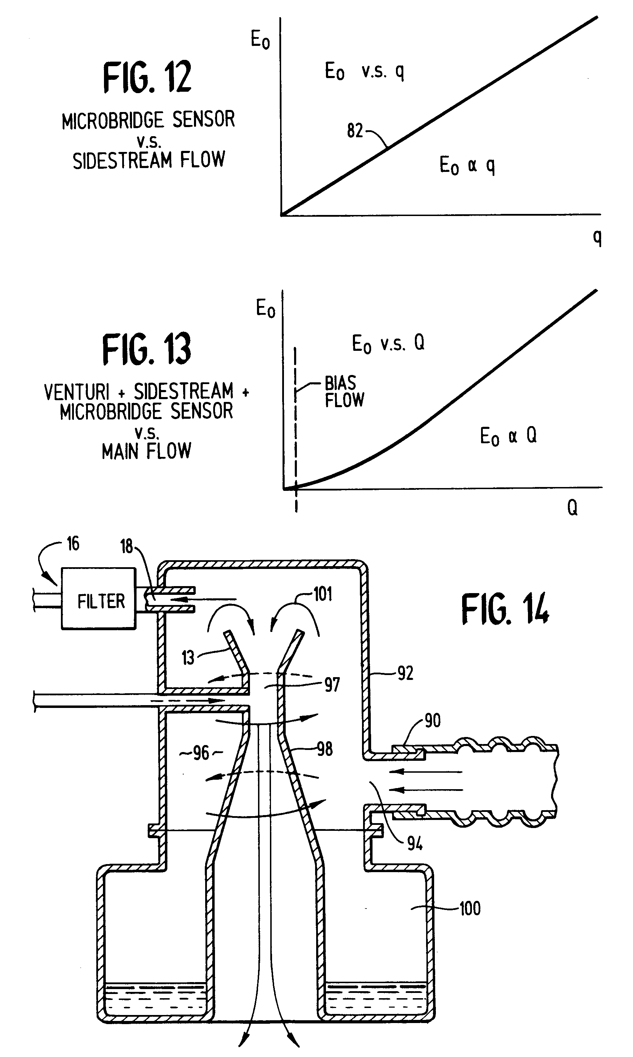

The flow sensor means 24 preferably comprises one or more sensors constructed so as to provide one or more output temperature compensated DC analog signals (Eₒ) representing the flow through the sidestream flow path. In a preferred embodiment, as is illustrated in Fig. 11, the sensor means 24 preferably comprises at least one miniaturized thermal bridge type linear mass flow sensor 74, such as a mass airflow integrated circuit chip sensor available, for example, under the trademark MICROBRIDGE from Microswitch, although other similar types of flow sensors may also be suitable. The bridge type flow sensor preferably includes a heater element 76 and first and second bridge temperature sensitive elements 78 and 80 such as thermistors, all disposed within the sidestream flow path. The first temperature sensitive element 78 upstream of the heater element is cooled by the airflow, and the second temperature sensitive element 80 downstream of the heater element is heated by heat from the heater element 76 carried by the airflow. Changes in temperature affect both elements substantially equally, and the bridge configuration of these elements is thus essentially responsive to the temperature differential between the temperature sensitive bridge elements. The bridge configuration of the temperature sensitive elements 78 and 80 thus provides a temperature compensated output DC analog signal linearly proportional to the mass airflow. This relationship is illustrated in Fig. 12 by curve 82. The bridge typically satisfies the following performance standards: a voltage of ten volts DC; a flow range of about zero to fifty mlpm linear, 200 mlpm maximum; an output of five volts DC at 200 mlpm and an operating temperature of zero to seventy degrees centigrade operating ambient with a humidity of about 85% max, throughout the air.

The flow sensor 74 is preferably a linear mass flow sensor with a low flow range of about zero to about 150 milliliters per minute ("mlpm"). Since the desired patient airflow range is to be measured up to 300 lpm, the precision venturi output scaling restrictions and the venturi restriction placed in the mainstream typically provide a resultant side flow of approximately 1/2000th of the main flow for measurement by the flow sensor. Associated electronic circuitry to the flow sensor, as shown in Fig. 11, provides amplification, a no-flow zero adjustment and a maximum-flow span adjustment for standardizing the analog signal. The accompanying bridge amplifier 84 preferably will have zero and span adjust potentiometers. These potentiometers allow adjustment of the signal to compensate for non-uniformity of the flow sensor 74, output scaling restrictors 66 and 68 and bias flow. They may be factory adjusted at zero and full scale flow. Readjustment should only be required if the bias flow system 54, sensor 74, or restrictors 66 or 68 are changed. The amplifier 84 is also provided to enable low pass filtering to obtain optimum noise rejection, with 15 ms response time.

The venturi method of generating side flow introduces some non-linearity so that linearization must be employed in subsequent signal conditioning. The output signal Eo of the flow sensor is then approximately proportional to patient airflow Q, as illustrated in Fig. 13. The classical method of linearization utilizing a conventional microprocessor, is to digitize the analog signal and use the digital value in a look-up table to determine a corresponding corrected digital value of the flow rate. The integral of the flow rate, typically calculated with a microprocessor, provides a measure of the patient tidal volume, which is the average volume of exhaled air per breath. Breathing rate (the average reciprocal of time between breaths), and minute volume (flow rate times tidal volume) are also determined by the microprocessor.

In an alternative preferred embodiment illustrated in Fig. 14, warm exhaled patient air is communicated through the external connecting conduit 90 into a coaxial venturi chamber 92. The warm, moisture laden air passes from the external connecting conduit through communicating port 94 into the interior 96 of the coaxial venturi chamber 92 to permit countercurrent heat exchange of the warm air with the typically relatively cooler mainstream conduit 13 and venturi orifice 97 disposed therein. As the interior coaxial mainstream venturi orifice 97 is warmed, the warm moist air is cooled, and moisture is thus condensed from the exhaled patient air impinging upon the exterior surface 98 of the venturi orifice 97. As a result, the condensation of moisture may collect upon the exterior surface to coalesce, and drip down to a container 100 depending from the bottom of the venturi chamber 92. The "dried" air, as indicated by arrows 101, passes through the interior 96 and up to the opening of the venturi mainstream conduit 13. The positive port 18 is maintained upstream of the orifice 40 for fluid communication with a sidestream flow subsystem 16. As with the previously described embodiment, the sidestream flow path exits into the most restricted part of the venturi orifice 97 for exhaust out of the system 10.

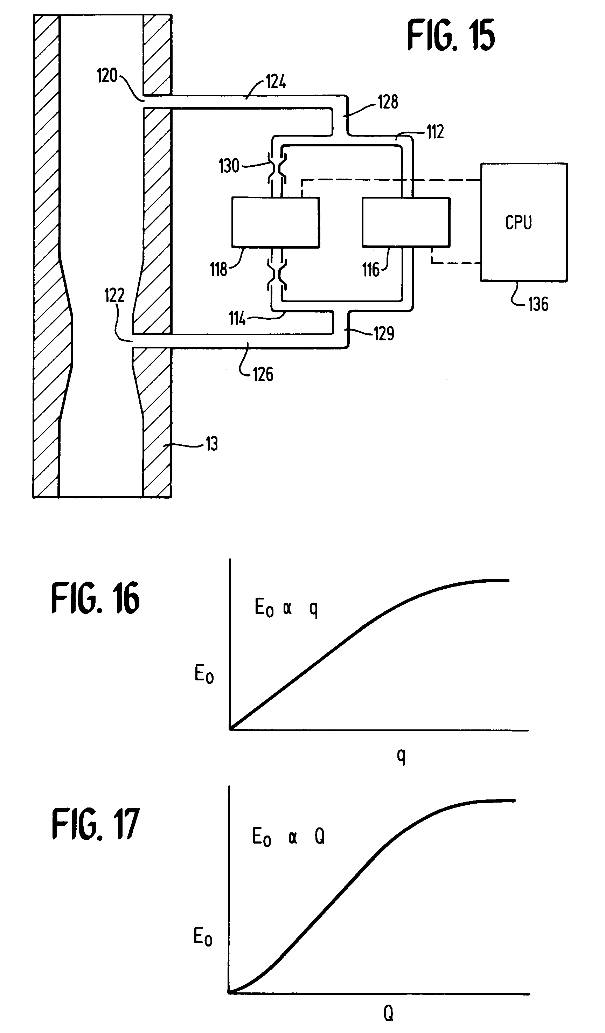

In another preferred embodiment illustrated in Fig. 15, the flow sensor subsystem 110 comprises a plurality of flow sensors in fluid communication with the mainstream conduit 13. The mainstream conduit 13 in this embodiment may be substantially horizontal or vertical. The flow sensor subsystem 110 includes at least first and second sidestream sensor conduits 112 and 114 connected for fluid communication with the mainstream conduit 13 and with first and second sensors 116 and 118, respectively, through positive and negative ports 120 and 122 and sidestream connecting tubing 124 and 126. The sidestream conduits 112 and 114 have a proximal end 128 in fluid communication with the mainstream conduit 13, upstream of the mainstream restrictor, e.g., the mainstream venturi 15, and a distal end 129 in fluid communication with the mainstream conduit downstream of the mainstream restrictor.

In this embodiment, a plurality of sidestream sensors having inlet and outlet connecting tubing of different sizes for each sensor to provide different flow rates to each sensor is utilized to maximize sensitivity and range of functional linearity of measured mass flow rates. By varying the sizing of sidestream conduits connected to each sensor, or use of differently sized sidestream restrictions for each sidestream conduit, each sidestream flow sensor will have a different sensitivity and linear range of functionality. Combining or selecting the signal outputs of sensors from these differently sized conduits or sidestream restrictions can provide a composite output having a broader linear functionality range than that of a single venturi conduit system. The CPU may be programmed to determine an approximate rate based upon signal outputs of one or more of the sensors and to select or switch to signal output from an individual search having a range of linear functionality encompassing the approximate flow rate, for determination of a more precise flow rate. Thus, by combining outputs from multiple sidestream conduits, each with a unique sensitivity and linearity range, the sensor subsystem 110 can be tuned to provide a more desirable response curve over selected mass flow rates.

For example, still referring to Fig. 15, an orifice restrictor 130 is formed or positioned within the second sidestream conduit 114. The first sidestream conduit 112, having no restrictor therein, has a mass flow sensitivity which is more sensitive and accurate, i.e., achieves linear functionality at lower mass flow rates, than the second sidestream conduit 114. First and second flow rate sensors 116 and 118, respectively, are disposed or positioned, respectively, within first and second sidestream conduits 112 and 114 and are electrically connected to conduct their output signals to the computer or central processing unit (CPU) 136. CPU 136 receives the output from both of the sensors, generally proportional to the mass flow rate through the mainstream conduit 13, to analyze and compare each signal relative a standard, usually by comparing a digital representation of each sensor's output to a look-up table, to ascertain which of the sensor's output is functionally more linear. After making the selection as to which Eₒ is the most linear, that output can then be manipulated to determine the mainstream flow rate.

Fig. 16 represents the generally linear proportional relationship between the temperature DC analog signal (Eₒ) and mass airflow of (q) the plural or multiple sidestream conduit system 110. As compared with Fig. 12, Fig. 16 illustrates an increased sensitivity and linearity of the DC analog signal at lower mass flow rates and insensitivity at higher mass flow rates for a net overall increase in the operational response range of a plural sensor subsystem. After linearization in subsequent signal conditioning, a composite response curve can be formed based upon Eₒ that is approximately proportional to mainstream patient airflow (Q), over a wider range of flow rates. Fig. 17 illustrates the difference in the responsiveness of a multiple sidestream conduit system as compared with the functionality of a single sidestream conduit system as shown in Fig. 13, particularly at low mainstream flow rates.

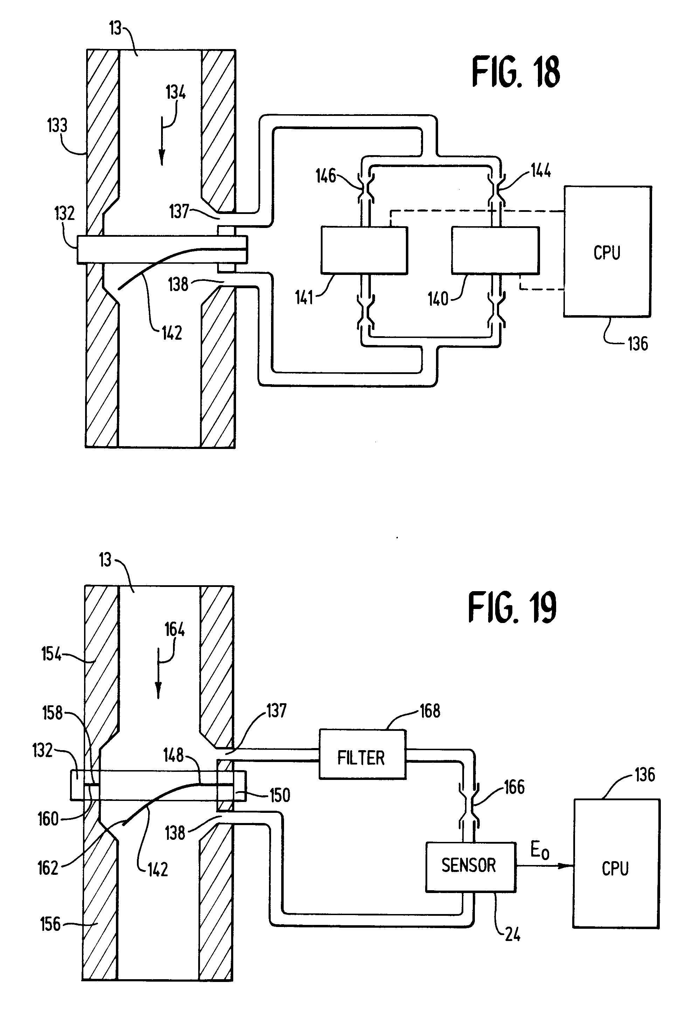

Referring now to Fig. 18, another embodiment of a spirometer in accordance with the present invention includes a variable orifice restrictor 132 formed within the mainstream conduit 13. Mainstream conduit 13 is formed within orifice body 133. The variable orifice restrictor 132 provides a variable backpressure to the airflow, indicated by arrow 134, through the mainstream conduit 13. Positive port 137 and negative port 138 are connected for fluid communication with sensor 140 and sensor 141 to provide signal outputs functionally related to patient airflow Q. The sensors 140 and 141 may advantaeously have differently sized output scaling restrictors 144 and 146, respectively, to provide different ranges of functional linearity of signal response. Alternatively, the multiple sensors may be replaced by a single sensor, as described earlier, to be used in combination with the mainstream variable orifice restrictor.

Referring now to Fig. 19, a variable orifice restrictor 132 identical to the restrictor shown in Fig. 18 extends substantially entirely across the mainstream conduit 13. In one embodiment, the variable orifice restrictor 132 includes a flexible flap 142 having a first end or base 148 mounted to one side 150 of the mainstream conduit 13. The mainstream conduit 13 may also include at least a first and second conduit portions 154 and 156 which terminate in a first and second juxtaposed surfaces 158 and 160. A second or distal portion 162 of the flap 142 extends substantially entirely across the mainstream conduit 13 and moves, under the influence of mass airflow, indicated by arrow 164, between a first position extending substantially transversely across the mainstream conduit with the distal end of the flap adjacent to the side of the conduit opposite the base of the flap, and a second position in which the distal end of the flap extends substantially downstream within the mainstream conduit and toward the side of the mainstream conduit to which the base of the flap is attached. This variable orifice restrictor 13 thus provides a variable backpressure. Sidestream subsystem 16, in fluid communication with the mainstream conduit 13, as more fully described elsewhere in this application, includes positive and negative ports 18 and 20, fluidly communicated by sidestream connecting tubing 64. Sensor 24 is fluidly connected to the mainstream conduit 13 through tubing 64 and positioned downstream of the output scaling restrictor 166 and filter 168 to provide an output Eₒ. The preferred scale of the sidestream flow is currently typically from about 1/1200th of the mainstream flow to about 1/12,000th of the mainstream flow, for use with the variable orifice type mainstream restrictor. Thus for the variable orifice restrictor the sidestream flow rate would be typically about 200 ml/min. for 40 l/min. flow in the patient airway for the low flow scale, and about 200ml/min. for 400 l/min. flow in the patient airway for the high flow scale.

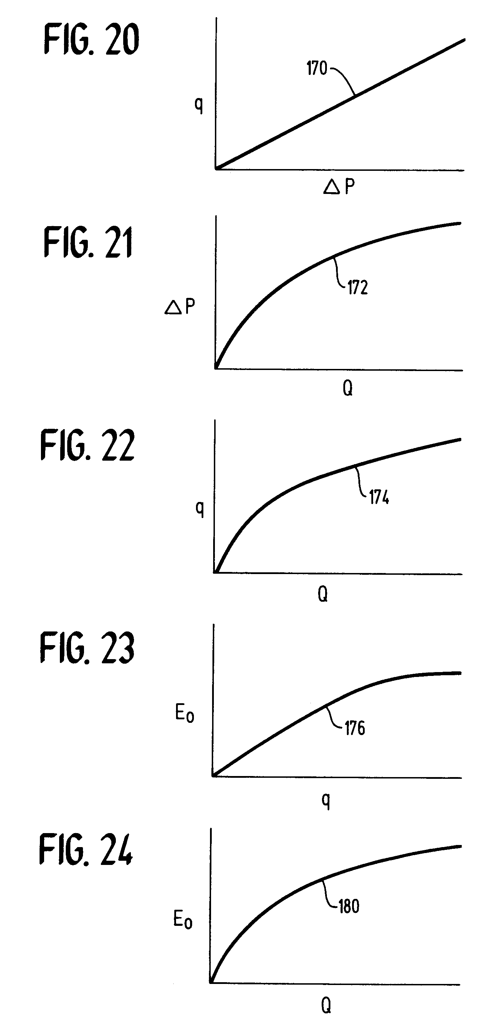

By this construction, the variable orifice system creates a sidestream flow ("q") generally proportional to the variable differential pressure ΔP. This is best understood by referring to Figure 20, which depicts curve 170 representing the relationship between sidestream flow q and the variable venturi differential pressure ΔP. However, as distinguished from the fixed restrictor venturi embodiments, the differential pressure ΔP will functionally vary with patient airflow due to the changing back pressure or resistance of the variable orifice with increased flow within the mainstream conduit. This relationship tends to become linearly proportional at higher patient airflow Q. This relationship is best understood by referring to Figure 21 which depicts curve 172 as representing the relationship between differential pressure ΔP and patient airflow Q.

Sidestream flow q, then, will be functionally related to mainstream flow rates through a variable restrictor orifice at lower patient airflow values, and will be generally directly proportional at higher airflow values. This relationship is best understood by referring to figure 21, which depicts curve 174 as representing the relationship between sidestream flow q and patient airflow Q. As sidestream mass airflow passes over the bridge elements of the flow sensor, the sensor generates a temperature compensated DC analog signal Eₒ which is linearly proportional to sidestream mass airflow q. This is best understood by referring to Fig. 23 which depicts curve 176 as representing the relationship between Eₒ and sidestream flow q. The flow sensor signal Eₒ is approximately proportional to the square root of the mainstream flow through a variable restrictor orifice, as is best understood with reference to Fig. 24, which depicts curve 180 as representing the relationship between output Eₒ and patient airflow Q. Thus, for a variable orifice restriction, the flow sensor signal Eₒ can be squared and scaled to yield the patient airflow rate Q. The square root curve illustrated in Fig. 24 provides enhanced sensitivity to low flow rate changes, and a look-up table can be used to provide precision squares determinations.

In summary, for all sidestream flow sensors utilized with a fixed mainstream venturi restriction, the relationship of the voltage of the sidestream sensor output signal Eₒ to the mainstream patient airflow is approximately linear. Therefore, one preferred method which could be implemented by the computer 136 in determining mainstream flow rate (Q) involves scaling the output signal Eₒ by a multiplicative factor (a) and an additive factor (b), where a and b can be determined through calibration of the sidestream flow sensor. Such a scaling method for determining mainstream flow rate according to the equation Q = aEₒ + b can alternatively be implemented in an electrical circuit. A computer or microprocessor is preferably used to determine the mainstream flow rate by selecting a flow rate value corresponding to the sensor output signal from a look-up table stored in a memory device of the computer or microprocessor, as explained above.

For sidestream flow sensors utilized with a variable mainstream orifice, the sidestream sensor signal output voltage Eₒ is approximately proportional to the square root of the mainstream flow (Q). A computer of microprocessor could similarly be implemented to determine the mainstream flow rate by determining the square of the sensor output voltage signal Eₒ, and scaling the value (Eₒ)² by a multiplicative factor a and an additive factor b, according to the equation Q = a(Eₒ)² + b, with a and b similarly being determined by calibration of the sensor. A look-up table is similarly preferably implemented by the computer of microprocessor to select a flow rate value corresponding to the sensor output signal voltage, as explained above.

It will be appreciated from the foregoing that the present invention represents a significant advance in the field of spirometry. In particular, embodiments of the present invention provide a sensitive, accurate and rugged method for measuring the flow rate of the particular ventilator. The newly developed sidestream flow sensor avoids problems asoociated with an obstruction in the airway by use of a restrictive venturi placed in the mainstream flow conduit. Furthermore, by having the venturi constructed to prevent condensation in the critical throat area without applied heat, the fluctuations in patient's respiration readings are minimized. Since the sensor 24 is located in a sidestream position, only filtering of the sidestream is required and not the mainstream, thereby reducing costs and flow resistance. The removable segment of the mainstream conduit containing the mainstream restriction and associated sidestream sensor means also allows for convenient maintenance of the sidestream flow sensor.

It will also be appreciated that, although presently preferred embodiments of the invention have been described by way of example, various modifications may be made without departing from the spirit and scope of the invention. Accordingly, the present invention is not to be limited except as by the appended claims.

## Claims

1. A flow sensing system suitable for measuring a flow rate of gas comprising:
a mainstream conduit adapted for carrying a flow of said gas therethrough and having means for restricting gas flow through said mainstream conduit for producing a pressure differential between an upstream portion of said gas flow on one side of said means for restricting gas flow and a downstream portion of said gas flow extending from within said means for restricting gas flow to the other side of said means for restricting gas flow;
a sidestream flow subsystem including at least one sidestream conduit having a cross-sectional area significantly less than said mainstream conduit and having a proximal end thereof connected to said mainstream conduit for fluid communication with said upstream portion of said gas in said mainstream conduit and a distal end connected to said mainstream conduit for fluid communication with said downstream portion of said gas in said mainstream conduit;
thermal flow sensing means disposed within each said sidestream conduit and operative to generate an output signal representing said sidestream flow rate; and
means connected to said thermal flow sensing means for determining the flow rate of gas within said mainstream conduit responsive to said output signal.

2. The flow sensing system of Claim 1, wherein said sidestream flow subsystem comprises first and second sidestream conduits, each of said sidestream conduits having flow rates significantly less than that of said mainstream conduit, and each of said sidestream conduits having a proximal end thereof in fluid communication with said mainstream conduit upstream from said means for restricting gas flow and a distal end in fluid communication with said mainstream conduit downstream of said means for restricting gas flow, said sidestream conduits having at least one restricted portion, and said restricted portions of said plurality of sidestream conduits being differently sized;
said thermal flow sensing means comprises first and second thermal flow sensors disposed within said first and second sidestream conduits, respectively, for generating output signals indicative of said gas flow rate through said mainstream conduit; and
means for selecting one of said output signals from said first and second flow sensors for use by said means for determining said flow rate of gas in said mainstream conduit.

3. A flow sensing system suitable for measuring a flow rate of gas comprising:
a mainstream conduit adapted for carrying a flow of said gas therethrough and having means for restricting gas flow through said mainstream conduit for producing a pressure differential between an upstream portion of said gas flow on one side of said means for restricting gas flow and a downstream portion of said gas flow extending from within said means for restricting gas flow to the other side of said means for restricting gas flow;
a sidestream flow subsystem including a plurality of sidestream conduits, each of said sidestream conduits having cross-sectional areas significantly less than that of said mainstream conduit, and each of said sidestream conduits having a proximal end thereof in fluid communication with said mainstream conduit upstream from said means for restricting gas flow and a distal end in fluid communication with said mainstream conduit downstream of said means for restricting gas flow, said sidestream conduits having at least one restricted portion, and said restrictions of said plurality of sidestream conduits being differently sized;
thermal flow sensing means disposed within each of said plurality of sidestream conduits operative to generate output signals indicative of said gas flow rate through said mainstream conduit;
means for selecting one of said output signals from said plurality of flow sensors for use by said means for determining said flow rate of gas in said mainstream conduit; and
means connected to said thermal flow sensing means for receiving said selected output signal and determining the flow rate of gas within said mainstream conduit responsive to said selected output signals.

4. The flow sensing system of any preceding claim, wherein said means for restricting gas flow is a fixed orifice restrictor.

5. The flow sensing system of Claim 4, wherein said means for restricting gas flow is a venturi having a narrowed throat formed within said mainstream conduit for restricting the flow of gas therethrough, and wherein said distal end of said sidestream conduit is connected to said venturi throat.

6. The flow sensing system of any of Claims 1 to 3, wherein said means for restricting gas flow is a variable orifice restrictor positioned within said mainstream conduit, and wherein said distal end of said sidestream conduit is connected to said mainstream conduit on said other side of said variable orifice restrictor.

7. The flow sensing system of Claim 6, wherein said mainstream conduit includes a first inner longitudinal side and a second inner longitudinal side substantially opposite said first side, said variable orifice restrictor includes a flap having a proximal base end and a distal end, said proximal base end flexibly is mounted to said first side of said mainstream conduit, and said flap is free to move between a first position extending generally transversely across said mainstream conduit and a second position in which said distal end extends generally downstream from said first position.

8. The flow sensing system of any preceding claim, wherein said gas is laden with moisture at an elevated temperature and said mainstream conduit is surrounded by an outer conduit arranged to direct said flow of moisture laden gas against said mainstream conduit so as to transfer heat to said mainstream conduit.

9. The flow sensing system of Claim 8, wherein said mainstream conduit is generally vertically disposed.

10. The flow sensing system of Claim 9, wherein said mainstream conduit has an outer surface, and further including receptacle means, said receptacle means depending from said outer conduit and in fluid communication with said exterior surface of said mainstream conduit, for receiving fluid therein.

11. The flow sensing system of any preceding claim, wherein said sidestream conduit includes a filter to remove contaminants from the gaseous stream.

12. The flow sensing system of any preceding claim, wherein said mainstream conduit includes a detachable segment, and the sidestream conduit is connected for fluid communication with said detachable segment of said mainstream conduit, whereby said detachable segment is easily removable from said mainstream conduit for cleaning and disposal.

13. The flow sensing system of any preceding claim, further including bias flow means, in fluid communication with said mainstream conduit, for producing a constant flow rate of air through said sidestream conduit.

14. The flow sensing system of any preceding claim, wherein said sidestream conduit includes a positive port at said proximal end, a negative port at said distal end, an input tubing connected to said positive port, an input restrictor disposed in said input tubing, an output tubing connected to said negative port, an output restrictor disposed in said output tubing, said input and output tubing being of equal length, and said input and output restrictors being symmetrically placed upstream and downstream, respectively, of said thermal flow sensing means.

15. The flow sensing system of any preceding claim, wherein said thermal flow sensing means comprises a heater element, a first temperature sensing element disposed upstream of said heater element, and a second temperature sensing element disposed downstream of said heater element, for measuring the flow rate of said gas through said sidestream conduit.

16. A method for measuring rate of gas flow in a mainstream conduit having a means for restricting gas flow therein, and a sidestream flow sensing subsystem connected for fluid communication with upstream and downstream portions of said gas flow relative to said means for restricting gas flow, said sidestream flow sensing subsystem having flow sensing means operative to generate an output signal representative of gas flow from said mainstream conduit through said sidestream flow sensing subsystem, said flow sensing means being operative to measure a range of sidestream gas flow rates over which the corresponding output signal is substantially linearly proportional to said sidestream gas flow, and comprising the steps of:
scaling said selected output signal by a multiplicative factor and an additive factor to generate an amplified sensor output signal; and
determining said gas flow rate in said mainstream conduit based upon said amplified sensor output signal.

17. The method of Claim 16, wherein said means for restricting gas flow in said mainstream conduit is a variable orifice restrictor, such that said sensor output signal is proportional to the square root of said mainstream gas flow rate, and said step of determining said mainstream gas flow rate comprises determining the square of said output signal, and scaling said squared output signal to determine said mainstream gas flow rate.

18. A method for measuring rate of gas flow in a mainstream conduit having a means for restricting gas flow therein, and a sidestream flow sensing subsystem connected for fluid communication with upstream and downstream portions of said gas flow relative to said means for restricting gas flow, said sidestream flow sensing subsystem having flow sensing means operative to generate an output signal representative of gas flow from said mainstream conduit through said sidestream flow sensing subsystem, said flow sensing means being operative to measure a range of sidestream gas flow rates over which the corresponding output signal is substantially linearly proportional to said sidestream gas flow, and comprising the steps of:
selecting the sensor output signal most substantially linearly proportional to said sidestream gas flow rate; and
determining said gas flow rate in said mainstream conduit based upon said selected sensor output signal.

19. The method of Claim 18, wherein said step of determining said gas flow rate in said mainstream conduit comprises scaling said selected output signal by a multiplicative factor and an additive factor to determine said mainstream gas flow rate.

20. The method of Claim 18, wherein said means for restricting gas flow in said mainstream conduit is a variable orifice restrictor, such that said sensor output signal is a function of said mainstream gas flow rate, and said step of determining said mainstream gas flow rate comprises determining the mainstream gas flow rate from said selected output signal.
